# EUROPÄISCHE PATENTSCHRIFT

(11) **EP 0 375 599 B1**
(45) Veröffentlichungstag und Bekanntmachung des Hinweises auf die Patenterteilung: **02.03.1994**
(21) Anmeldenummer: 89730225.3
(22) Anmeldetag: 20.12.1989
(51) Int. Cl.: A61F 2/30

(54) **Endoprothese**
Endoprosthesis
Endoprothèse

(30) Priorität: 23.12.1988 DE 3844157
(43) Veröffentlichungstag der Anmeldung: 27.06.1990
(73) Patentinhaber: Johnson & Johnson Professional Products GmbH, 22848 Norderstedt (DE)
(72) Erfinder: Kranz, Curt, Dr., D-1000 Berlin 62 (DE)
(74) Vertreter: UEXKÜLL & STOLBERG

(56) Entgegenhaltungen:
- EP-A- 0 289 922
- DE-A- 2 933 237
- DE-A- 2 941 265
- Dubbel Taschenbuch für den Maschinenbau, 15. Auflage, Seiten 1070 und 1071; 17. Auflage, Seite C7

## Beschreibung

Die Erfindung betrifft eine Endoprothese der im Oberbegriff des Anspruchs 1 angegebenen Art.

Prothesenschäfte werden bei der Implantation in den passend vorgeraspelten Knochenhohlraum fixiert, z.B. einzementiert oder für zementfreie Implantation zum gelenkfernen Schaftende hin konisch oder keilartig ausgeführt und in den angepaßten Hohlraum eingeschlagen, so daß sie durch Preßsitz mit dem Knochen verbunden sind.

Aus der DE-A-29 33 237 ist eine Prothese der eingangs genannten Gattung bekannt, die bezüglich ihrer lokalen Elastizität und Steifigkeit an die umgebenden Knochenbereiche angepaßt ist. Diese Anpassung bezieht sich aber lediglich auf die Querschnittsabmessungen und ihre Abstimmung auf in Richtung der Längsachse wirkende Kräfte. Querschnitt und Wandstärke nehmen vom oberen zum unteren Schaftende hin kontinuierlich ab. Nachteilig ist dabei, daß die bei außerhalb der Längsachse angreifenden Kräften auftretenden Verformungen sich nicht konform zu den ensprechenden Verformungen der angrenzenden Knochenbereiche verhalten. Mikrobewegungen und eine mögliche Lockerung der Prothese sind die Folge.

Aus der EP-A-0 243 585 ist weiterhin ein Prothesenschaft mit im wesentlichen rechteckigen Querschnitt bekannt. An den die beiden längeren Seiten des rechteckförmigen Querschnitts (dorsal und ventral) bildenden Flächenteilen sind Durchbrüche vorgesehen, die den Schub, welcher bei der durch Belastung verursachten Biegung an der Grenzfläche zwischen dem Knochen und dem implantierten Schaft auftritt, herabsetzen. Nachteilig bei diesem Schaft ist vor allem die relativ hohe Flächenpressung zwischen Schaft und Knochen im Bereich der beiden kürzeren Rechteckseiten (medialen und lateral). Außerdem fehlt es wegen der geometrisch-rechteckigen Querschnittsform des Schaftes an einer physiologischen Anpassung an den natürlichen Markraum, wodurch es zu Kraftspitzen im Bereich des Adamschen Bogens kommt. Die links- und rechtsseitigen unterschiedlichen Geometrien des Femurmarkraumes finden dabei ebenfalls keine Berücksichtigung. Zwar ist die Biegsamkeit der Prothese durch in einer parallel zur Hauptkrümmungsebene gelegenen Ebene angeordnete Aussparungen heraufgesetzt - wie es auch schon bei der aus der vorgenannten Vorveröffentlichung bekannten Prothese der Fall war - jedoch entspricht diese Biegsamkeit nicht derjenigen des umgebenden Knochenraums.

Der Erfindung liegt die Aufgabe zugrunde, bei einer Endoprothese der eingangs genannten Gattung die Anpassung der Verformbarkeit des Schaftes an die entsprechenden Eigenschaften der kortikalen Röhre des Knochens dadurch zu verbessern, daß die lokalen Biegeeigenschaften denjenigen des Röhrenknochens möglichst weitgehend angepaßt werden.

Die Erfindung beruht auf der Erkenntnis, das alle bekannten Endoprothesen, mit Ausnahme der isoelastischen Prothesen, eine im Vergleich zum kortikalen Knochenrohr wesentlich höhere Biege- und Längssteifigkeit aufweisen, wodurch es zu einer Entlastung der Kortikalis (sogenannte Stress-Shielding) kommt. Der Steifigkeitssprung am gelenkfernen Schaftende führt zu einer Unstetigkeit in der Spannungsverteilung. Der sprunghafte Anstieg der Längsspannung bewirkt durch Reizung der Kortikalis eine verstärkte Kallusbildung. Diese Sockelbildung stellt jedoch ein Hindernis für ein geringfügiges postoperatives Einsinken und für eine eventuell notwendige nachträgliche Neuverankerung des Schaftes dar. Außerdem nimmt dadurch die Abweichung von physiologisch normalen Verhältnissen immer mehr zu. Bei der erfindungsgemäßen Prothese wird dagegen durch die geringe Biegesteifigkeit des gelenkfernen Schaftes der Spannungssprung vermieden. Trotzdem ist eine ausreichende Biege- und Längssteifigkeit im gelenknahen Bereich gewährleistet.

Es wurde gefunden, daß durch eine nichtlinear abnehmende Biegesteifigkeit und eine annähernd linear abnehmende Längssteifigkeit vom gelenknahen zum gelenkfernen Ende des Schaftes eine gleichmäßige und physiologisch günstige Krafteinleitung in den Knochen erzielbar ist. Die Biegesteifigkeit nimmt vom gelenknahen zum gelenkfernen Ende des Schaftes zunächst stark ab, um im gelenkfernen Teil (etwa über die restliche halbe Schaftlänge) nur noch langsam abzusinken. Unstetigkeiten werden damit sowohl in bezug auf die Biegesteifigkeit als auch in bezug auf die Längssteifigkeit vermieden. Bevorzugt wird eine zum gelenkfernen Ende hin verjüngte Hohlrohrform mit gleichmäßig abnehmender Wandstärke gewählt, wobei der außen gebogene, laterale Halbrohrbereich am gelenkfernen Ende über den innen gelegenen medialen Halbrohrbereich hinausragt. Im Bereich des gelenkfernen Endes, das durch Entfernen eines im wesentlichen halbrohrförmigen Bereichs selbst nur noch halbrohrförmig ausgebildet ist, hat die Biegesteifigkeit, in die das Flächenträgheitsmoment eingeht, bereits stark abgenommen, während die Längssteifigkeit, die vom Materialquerschnitt bestimmt wird, nur geringfügig abnimmt.

Durch die lokale Anpassung der Biegesteifigkeit des Prothesenschaftes an den umgebenden Knochen wird weiterhin erreicht, daß die Längssteifigkeit des Schaftes entsprechend dem Prinzip der angepaßten Verformungen eine zum gelenkfernen Ende hin stark zunehmende Fähigkeit aufweist, der Biegung des Röhrungknochens unter Belastung zu folgen. Das entspricht einer sogenannten Schäftung.

Im Falle einer Hüftgelenkendoprothese wird die Biegesteifigkeit im proximalen Bereich mindestens soweit aufrecht erhalten werden, wie es nötig ist, eine Querkraftentlastung des Adamschen Bogens zu leisten. In diesem Bereich werden während der Implantation die stützenden Spongiosastege aus dem Markraum entfernt, die vorher die Querkraftentlastung durch Schubübertragung übernehmen konnten. In Richtung zum gelenkfernen Ende des Hohlschaftes muß die Biegesteifigkeit des Schaftes abnehmen. Eine derartige Konstruktion kann durch ein Rohr, welches eine sich kontinuierlich ändernde Wanddicke aufweist, relativ einfach verwirklicht werden. Auch gezielt angebrachte Wanddurchbrüche und Vertiefungen innerhalb der Wandung können diesen Effekt hervorrufen bzw. verstärken.

Allerdings sind dabei Wanddurchbrüche bevorzugt nicht an der konvexen, in der maximalen Krümmung nach außen weisenenden Seite des Schaftes anzubringen, da die Zugspannungen in diesem Bereich zu groß sind. Da andererseits auch an Bohrungen oder anders gearteten Durchbrüchen Kerbspannungen entstehen können, wodurch die Ermüdungsfestigkeit der Prothese herabgesetzt wird, sind Durchbrüche bevorzugt (parallel zur Ebene der maximalen Verformung vorzusehen) zusätzlich zu der sich über die Schaftlänge erstreckenden Wandquerschnittabnahme vorzusehen, wenn die hierdurch erzielbare lokale Steifigkeitsanpassung nicht ausreichend ist.

Während Aussparungen, die parallel zur Ebene der maximalen Krümmung des Prothesenschafts angeordnet sind, die Biegesteifigkeit im Bereich des Adamschen Bogens herabsetzen und hier eine Anpassung an die Biegesteifigkeit des Knochenmaterials erreicht wird, ist die Anpassung an einen idealisierten, den entsprechenden Eigenschaften des umgebenden Knochenmaterials angepaßten Verlauf der Längssteifigkeit durch die erfindungsgemäßen Maßnahmen und insbesondere durch Ausnehmungen, welche sich zum Schaftende hin erstrecken, insbesondere die Verkürzung der Innenseite des Hohlschaftes am gelenkfernen Ende, verbessert. In diesem Bereich ist eine geringe, kaum noch abnehmende Biegesteifigkeit ausreichend.

Aufgrund ihrer Eigenschaften, wie guter Biokompatibilität, hoher Ermüdungsfestigkeit und guter Korrossionsbeständigkeit, ist die Titan-Aluminium-Vanadium-Schmiedelegierung (TiAl6V4) als Werkstoff zur Herstellung von Prothesenschäften besonders geeignet. Dieses Material ist allerdings nicht gießbar. Die Verarbeitung erfolgt demzufolge durch Schmiede- oder Preß- und Schweißverfahren. Der Hohlschaft läßt sich aus zwei gepreßten oder geschmiedeten Halbschalen zusammenschweißen.

Die erfindungsgemäße Endoprothese ist bevorzugt kragenlos ausgebildet, da eine Einpassung für Krafteinleitung über Kragen und Schaft zugleich, unter Operationsbedingungen nur unter hohem Aufwand zu erreichen ist. Ein Aufsitzen des Kragens allein, also ohne entsprechenden Paßsitz des Schaftes kann dabei zu Resorptionserscheinungen am Adamschen Bogen und damit zu einer Lockerung der Prothese führen.

Der Halsbereich der Prothese ist insbesondere mit einem Steckkonus versehen, damit Kugeln unterschiedlichen Durchmessers und mit verschiedenen Konusstecklängen verwendet werden können.

Da Durchbrüche in der Wandung des Hohlschaftes dazu führen können, daß Knochensubstanz in den Hohlraum des Schaftes hineinwächst und ein derartiger Formschluß das Entfernen auch einer eventuell gelockerten Prothese, erschwert, sind gemäß vorteilhafter Weiterbildungen der Erfindung Maßnahmen vorgesehen, welche entweder das Einwachsen verhindern oder das Entfernen des eingewachsenen Knochens ermöglichen.

Ersteres erfolgt bevorzugt dadurch, daß der Prothesenhohlraum vollständig mit Polyethylen o.ä. aufgefüllt wird. Die geringere Steifigkeit des Polyethylens steht dabei den Elastizitäts- und Steifigkeitsanforderungen nicht entgegen. Eine günstige Weiterbildung, die darin besteht, daß die Polyethylenoberflächen im Bereich der Durchbrüche mit Apatit beschichtet sind, verhindert zudem, daß das Polyethylen direkt mit dem Knochen in Kontakt tritt.

Eine weitere vorteilhafte Möglichkeit lokale Wandabschwächungen zu realisieren, besteht darin, Vertiefungen in der Hohlschaftwandung vorzusehen. Wird der Hohlschaft aus mehreren Teilen zusammengesetzt und ist dadurch die innere konkav gewölbte Oberfläche vor dem Zusammenfügen zugänglich, können derartige Vertiefungen günstigerweise an den Innenflächen des Schaftes vorgesehen werden. Andernfalls besteht die Möglichkeit, Vertiefungen auch an der konvex gewölbten Oberfläche vorzusehen. Diese müssen jedoch mit einem geeigneten Material, beispielsweise Porocoat, aufgefüllt werden, um das Losbrechen des eingewachsenen Knochens im Falle einer Explantation zu erleichtern.

Eine weitere Variante, bei Vorhandensein von Durchbrüchen eine Explantation zu ermöglichen, besteht darin, den Prothesenhals so auf der Prothese zu fixieren, daß der Chirurg bei einer Explantation des Schaftes in der Lage ist, diesen zunächst vom Schaft zu trennen. Der dann vor ihm liegende offene Hohlraum des Schaftes ermöglicht es, den eventuell in den Hohlraum hineingewachsenen Knochen mit Fräs- oder Bohrwerkzeugen zu entfernen, ohne die Kortikalis zu beschädigen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein Ausführungsbeispiel der erfindungsgemäßen Hohlschaftprothese mit Scheibe und Steckkonus,
Figur 2 einen Längsschnitt durch den Hohlschaft der Prothese gemäß Figur 1,
Figur 3 eine Variante des in Figur 2 dargestellten Hohlschafts, der Durchbrüche aufweist,
Figur 4 einen Längsschnitt durch eine weitere Variante eines Hohlschafts, der Vertiefungen an der Innenseite der Wandung aufweist,
Figur 5 einen Längsschnitt durch eine weitere Variante eines Hohlschafts, der Vertiefungen an der Außenseite der Wandung aufweist,
Figur 6 einen Schnitt durch eine Variante der erfindungsgemäßen Hohlschaftprothese mit entfernbarem Kopfteil sowie
Figuren 7 und 8 vergrößerte Detailwiedergaben von Varianten des Ausführungsbeispiels im Querschnitt.

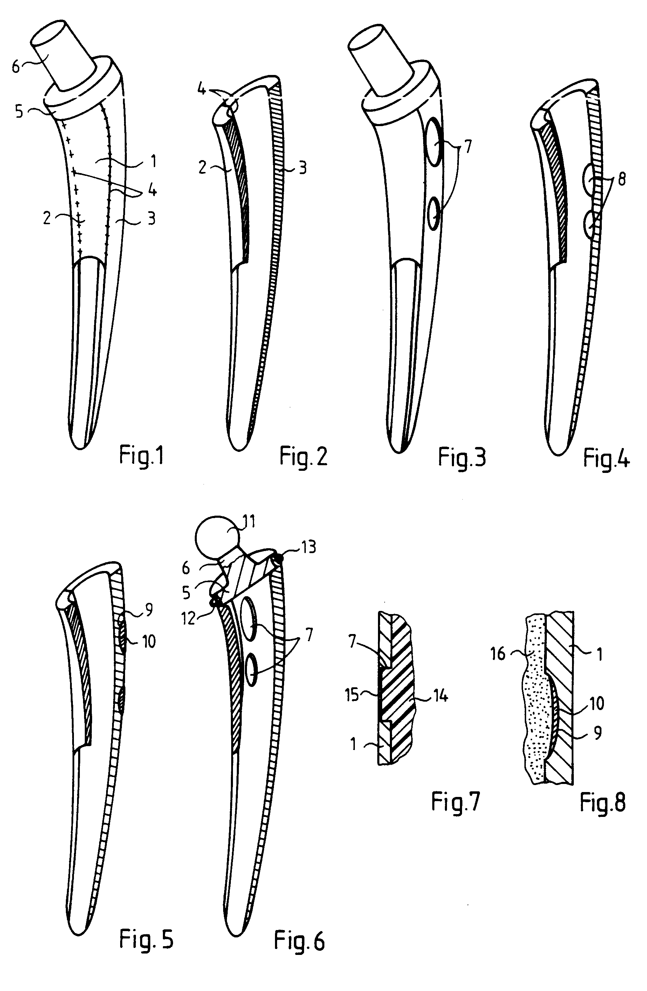

Ein Hohlschaft 1 des in Figur 1 dargestellten Ausführungsbeispiels der erfindungsgemäßen Hohlschaftprothese besteht aus zwei gepreßten oder geschmiedeten und miteinander verschweißten Halbschalen 2 und 3. Dabei bildet eine Halbschale 2 den inneren, medialen Teil und die andere Halbschale 3 den äußeren, lateralen Teil des Schaftes 1. Der laterale Teil ragt am gelenkfernen Ende im Bereich etwa der halben Schaftlänge über den medialen Teil hinaus. Das bedeutet auch, daß sich eine die beiden Halbschalen 2 und 3 verbindende Schweißnaht 4 nicht über die gesamte Länge des Schaftes 1 zu erstrecken braucht, womit einer Vereinfachung bei der Herstellung verbunden ist. Am gelenknahen Ende ist der Schaft 1 mit einer Scheibe 5 verschweißt, welche einen Steckkonus 6 zur Aufnahme verschiedener mit einem entprechenden Innenkonus versehener Gelenkkugeln trägt.

Die äußere Form des Schaftes 1 entspricht etwa der eines leicht gebogenen konischen Rohres und ist damit an den natürlichen Markraum des Femurs angepaßt. Die Konfektionierung der Schäfte ergibt sich aus einer großen Anzahl computertomographischer Messungen. Um insbesondere Torsionsbelastungen abfangen zu können, erweist es sich als günstig, die rechts- und linksunterschiedlichen Geometrien des Femurmarkraumes zu berücksichtigen und rechts- und linksseitig angepaßte Prothesen vorzusehen, welche eine zusätzliche Formgebung aufweisen, die an sich bekannt ist. Die in den Figuren dargestellten symmetrisch ausgebildeten Ausführungsbeispiele sind für beidseitige Anwendungen vorgesehen.

In Figur 2 ist ein Längsschnitt durch den Schaft 1 gemäß Figur 1 dargestellt. Es ist erkennbar, daß die Wanddicke des Schaftrohres vom gelenknahen zum gelenkfernen Ende abnimmt. Die stetige und kontinuierliche Variation der Wanddicke ermöglicht eine sehr weitgehende Anpassung der Längssteifigkeit des Prothesenschaftes an die des Knochenrohres, wobei Spannungsspitzen vermieden werden und die Anlagefläche des Schaftes an den Knochen unbeeinflußt bleibt.

Der in Figur 3 dargestellte Prothesenschaft weist dagegen einige große Durchbrüche 7 auf. Diese Durchbrüche dienen der Verstärkung des oben geschilderten Effektes. Grundsätzlich sind auf der nach außen gebogenen, lateralen Seite des Schaftes keine Durchbrüche anzubringen, da dieser äußere Teil des Prothesenschaftes sehr stark zugbeansprucht wird. Da an Bohrungen oder anderen Durchbrüchen Kerbspannungen entstehen, die bezüglich der Dauerfestigkeit der Prothese zu Problemen führen können sind bevorzugt nur wenige, entsprechend größer, dimensionierte Durchbrüche vorgesehen. Diese verringern die Biegesteifigkeit der Prothese im Bereich ihrer größten Krümmung.

Da solche Durchbrüche aber dazu führen können, daß Knochen in den Schafthohlraum hineinwächst, womit eine eventuell notwendige Explantation erheblich erschwert wird, ist es günstig, entweder das Einwachsen selbst zu verhindern oder aber das Gelenkteil vom Schaft abnehmbar auszuführen. Letzteres ermöglicht das spätere Entfernen des eingewachsenen Knochens vom Inneren des Schaftes her, insbesondere durch Ausbohren.

Das Einwachsen der Spongiosa in den Schaft wird bei dem in Figur 3 dargestellten Ausführungsbeispiel durch Auffüllen des Schafthohlraumes mit Polyethylen 14 und Versehen der Polyethylenoberfläche innerhalb der Durchbrüche 7 mit einer Beschichtung 15 aus Apatit verhindert werden. Eine derartige Ausführungsvariante ist in Figur 7 vergrößert wiedergegeben.

Eine andere Möglichkeit, gezielt Wandabschwächungen zu erzeugen, besteht darin, die innere Oberfläche, wie in der Schnittdarstellung von Figur 4 ersichtlich, mit Vertiefungen 8 zu versehen. Die Einbringung solcher Vertiefungen 8 ist allerdings nur möglich, wenn die innere Oberfläche zugänglich ist. Das ist insbesondere bei einem zweistufigen Herstellungsverfahren für Prothesenschäfte möglich, bei dem zunächst zwei gepreßte oder geschmiedete Halbschalen vorhanden sind, die erst in einem späteren Verarbeitungsschritt miteinander verbunden werden. Diese Halbschalen lassen sich, soweit das durch die Preß- oder Schmiedeumformung noch nicht geschehen ist, durch spanabhebende Verfahren, beispielsweise auch durch chemisches Fräsen, bearbeiten.

Die Einbringung von Vertiefungen 9 in die äußere Oberfläche - vgl. Figur 5 - ist dagegen generell möglich. Um ein Einwachsen von Knochen in die Vertiefungen 9 zu verhindern, sind die Vertiefungen 9 mit Porocoat-Material 10 aufgefüllt.

Bei einer in Figur 8 vergrößert wiedergegebenen Variante sind die Vertiefungen 9′ nicht völlig mit der Porocoat-Beschichtung 10 ausgefüllt, so daß eine Einsenkung im Vergleich zu benachbarten Bereichen der Schaftoberfläche verbleibt. Das in die verbleibende Einsenkung einwachsende Knochenmaterial 16 sichert durch Verbindung mit der Porocoat-Beschichtung einen festen Sitz des Schaftes. Sollte jedoch später einmal ein Entfernen des Schaftes notwendig werden, so "schert" die in die Einsenkung eingewachsne Knochensubtanz ab und gibt die Prothese frei, da die durch Abscheren entstandene Bruchfläche der Knochensubstanz in etwa konform mit den benachbarten Bereichen der Schaftoberfläche verläuft und keine wesentliche Sperre bildet. Die Grenzfläche, zwischen Knochen und Porocoatmaterial, die eine besonders feste Verbindung bildet, braucht dagegen nicht gelöst zu werden.

Bei im Prothesenschaft vorgesehenen Durchbrüchen besteht auch die Möglichkeit, den Knochen ungehindert in den Schafthohlraum einwachsen zu lassen, um ihn bei einer eventuell notwendigen Explantation im Rahmen einer Reoperation vom Innenraum der Prothese her auszubohren. Das ist aber nur dann möglich, wenn der Schafthohlraum im Bedarfsfall zugänglich ist. In diesem Fall ist eine lösbare Verbindung zwischen Gelenkteil 11 und dem Hohlschaft 1 vorgesehen. Eine derartige lösbare Verbindung zwischen Hohlschaft 1 und Gelenkteil 11 ist in Figur 6 dargestellt. Für die Implantation wird die Scheibe 5 des Gelenkteiles 11 auf das oberseitig flanschartig geformte Ende des Hohlschaftes 1 aufgesteckt und mittels eines äußeren Spannringes 12 fixiert. Der Spannring 12 weist eine Sollbruchstelle 13 in Form einer Materialschwächung auf, so daß der Gelenkteil bei Bedarf nach Lösen des Spannringes mit geringer Kraftanstrengung wieder entfernbar ist.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Endoprothese, insbesondere Hüftgelenkendoprothese, mit einem in einen Knochenhohlraum implantierbaren rohrförmigen, gebogenen und sich bezüglich Durchmesser und Materialquerschhitt verjüngenden Hohlschaft mit vom gelenknahen zum gelenkfernen Ende hin abnehmender Biegesteifigkeit,
**dadurch gekennzeichnet,**
daß die Biegesteifigkeit des Schaftes vom gelenknahen zum gelenkfernen Ende hin relativ starker abnimmt als die Längssteifigkeit.

2. Endoprothese nach Anspruch 1, **dadurch gekennzeichnet,** daß zur Verringerung der Biegesteifigkeit mindestens ein sich bis zum gelenkfernen Ende hin erstreckender Bereich ausgespart ist.

3. Endoprothese nach Anspruch 2, **dadurch gekennzeichnet,** daß der ausgesparte Bereich an der Innenseite der Krümmung d.h.medial, gelegen ist.

4. Endoprothese nach Anspruch 3, **dadurch gekennzeichnet,** daß der verbleibende Schaftbereich im wesentlichen halbrohrförmig ausgebildet ist.

5. Endoprothese nach einem der Ansprüche 3 oder 4, **dadurch gekennzeichnet,** daß der ausgesparte Bereich schlitzförmig ausgebildet ist.

6. Endoprothese nach Anspruch 4, **dadurch gekennzeichnet,** daß bei dem aus zwei Halbrohren zusammengefügten Hohlschaft das, bezogen auf die Krümmung des Schaftes, äußere (laterale) Halbrohr (3) das innere (mediale) Halbrohr (2) am gelenkfernen Ende überragt.

7. Endoprothese nach einem der vorangehenden Ansprüche, **dadurch gekennzeichnet,** daß der Schaft formmäßig links- oder rechtseitig angepaßt ausgebildet ist.

8. Endoprothese nach einem der vorangehenden Ansprüche, **dadurch gekennzeichnet,** daß der Hohlschaft (1) aus Titan-Aluminium-Vanadium-Schmiedelegierung (TiAl6V4) besteht.

9. Endoprothese nach einem der vorangehenden Ansprüche, **dadurch gekennzeichnet,** daß in der Wandung des Hohlschaftes (1) Aussparungen in Form von Durchbrüchen (7) vorgesehen sind.

10. Endoprothese nach Anspruch 9, **dadurch gekennzeichnet,** daß der Hohlraum des Schaftes (1) mit Polyethylen gefüllt und die Polyethylenoberfläche im Bereich der Durchbrüche (7) mit Apatit beschichtet ist.

11. Endoprothese nach Anspruch 9, **dadurch gekennzeichnet,** daß ein einen Teil eines Gelenkes aufnehmendes oder aufweisendes Teil (11) mittels eines, eine Sollbruchstelle aufweisenden Klemmringes (12) mit dem gelenknahen Ende des Hohlschaftes (1) lösbar verbunden ist.

12. Endoprothese nach einem der vorangehenden Ansprüche, **dadurch gekennzeichnet,** daß Vertiefungen (8 oder 9) in der Wandung des Hohlschaftes (1) als Wandschwächungen vorgesehen sind.

13. Endoprothese nach einem der vorangehenden Ansprüche, **dadurch gekennzeichnet,** daß mindestens ein Teil der Oberfläche mit Porocoat-Material überzogen ist.

14. Endoprothese nach den Ansprüchen 12 und 13, **dadurch gekennzeichnet,** daß die nach außen weisenden Vertiefungen (9) mit Porocoat-Material (10) gefüllt sind.

15. Endoprothese nach Anspruch 14, **dadurch gekennzeichnet,** daß die nach außen weisenden Vertiefungen nur zum Teil mit Porocoat-Material ausgefüllt sind, so daß die Oberfläche der mit dem Porocoat-Material versehenen Vertiefung eine Einsenkung in Bezug auf benachbarte Oberflächenbereiche des Prothesenschafts bildet.

16. Endoprothese nach einem der vorangehenden Ansprüche, **dadurch gekennzeichnet,** daß der Schaft kragenlos ausgebildet ist.

17. Endoprothese nach Anspruch 11, **dadurch gekennzeichnet,** daß das Gelenkteil (11) einen Steckkonus (6) aufweist.

## Claims

1. Endoprosthesis, in particular a hip joint endoprosthesis, with a hollow shaft which can be implanted in a bone cavity and which is tubular, curved and tapering in terms of diameter and material cross-section, the said shaft having a bending rigidity decreasing from the end near the joint to the end remote from the joint, characterized in that the bending rigidity of the shaft decreases from the end near the joint to the end remote from the joint to a relatively greater extent than does the longitudinal rigidity.

2. Endoprosthesis according to Claim 1, characterized in that, in order to reduce the bending rigidity, at least one area is hollowed out and extends as far as the end remote from the joint.

3. Endoprosthesis according to Claim 2, characterized in that the hollowed-out area is situated on the inner side of the curvature, i.e. medially.

4. Endoprosthesis according to Claim 3, characterized in that the remaining shaft area is of essentially semi-tubular design.

5. Endoprosthesis according to either of Claims 3 or 4, characterized in that the hollowed-out area is of slot-shaped design.

6. Endoprosthesis according to Claim 4, characterized in that, in the hollow shaft joined together from two half tubes, the outer (lateral) half tube (3), with respect to the curvature of the shaft, extends beyond the inner (medial) half tube (2) at the end remote from the joint.

7. Endoprosthesis according to one of the preceding claims, characterized in that the shaft is designed so as to be adapted in shape to be left-sided or right-sided.

8. Endoprosthesis according to one of the preceding claims, characterized in that the hollow shaft (1) consists of titanium/aluminium/vanadium wrought alloy (TiAl6V4).

9. Endoprosthesis according to one of the preceding claims, characterized in that recesses in the form of openings (7) are provided in the wall of the hollow shaft (1).

10. Endoprosthesis according to Claim 9, characterized in that the hollow area of the shaft (1) is filled with polyethylene, and the polyethylene surface is coated with apatite in the area of the openings (7).

11. Endoprosthesis according to Claim 9, characterized in that a part (11) receiving or having a part of a joint is detachably connected to the end of the hollow shaft (1) near the joint by means of a clamping ring (12) which has a predetermined breaking point.

12. Endoprosthesis according to one of the preceding claims, characterized in that depressions (8 or 9) are provided in the wall of the hollow shaft (1) as wall-weakening areas.

13. Endoprosthesis according to one of the preceding claims, characterized in that at least part of the surface is coated with Porocoat material.

14. Endoprosthesis according to Claims 12 and 13, characterized in that the outward facing depressions (9) are filled with Porocoat material (10).

15. Endoprosthesis according to Claim 14, characterized in that the outward facing depressions are filled only partly with Porocoat material, so that the surface of the depression provided with the Porocoat material forms an indentation in relation to adjacent surface areas of the prosthesis shaft.

16. Endoprosthesis according to one of the preceding claims, characterized in that the shaft is of a collarless design.

17. Endoprosthesis according to Claim 11, characterized in that the joint part (11) has an attachment cone (6).

## Revendications

1. Endoprothèse, notamment endoprothèse d'articulation de hanche, comprenant une tige creuse tubulaire, implantable dans une cavité osseuse, incurvée et effilée en diamètre et en section de matière, et dont la rigidité de flexion décroît de l'extrémité proche de l'articulation à l'extrémité éloignée de l'articulation,
caractérisée
en ce que la rigidité de flexion de la tige décroît plus fortement que la rigidité longitudinale, de l'extrémité proche de l'articulation à l'extrémité éloignée de l'articulation.

2. Endoprothèse selon la revendication 1, caractérisée en ce que, pour réduire la rigidité de flexion, au moins une région s'étendant jusqu'à l'extrémité éloignée de l'articulation est enlevée.

3. Endoprothèse selon la revendication 2, caractérisée en ce que la région enlevée est située sur le côté intérieur de la courbure, c'est-à-dire en position médiane.

4. Endoprothèse selon la revendication 3, caractérisée en ce que la région restante de la tige est de configuration sensiblement semi-tubulaire.

5. Endoprothèse selon une des revendications 3 et 4, caractérisée en ce que la région enlevée est en forme de fente.

6. Endoprothèse selon la revendication 4, caractérisée en ce que, dans le cas où la tige creuse est formée par assemblage de deux demi-tubes, le demi-tube (3) qui est extérieur par rapport à la courbure de la tige (demi-tube latéral) déborde au-delà du demi-tube (2) qui est intérieur (demi-tube médian), à l'extrémité éloignée de l'articulation.

7. Endoprothèse selon une des revendications précédentes, caractérisée en ce que la tige est d'une conformation adaptée pour le côté gauche ou pour le côté droit.

8. Endoprothèse selon une des revendications précédentes, caractérisée en ce que la tige (1) est composée d'un alliage de forge titane-aluminium-vanadium (TiAl6V4).

9. Endoprothèse selon une des revendications précédentes, caractérisée en ce que des évidements présentant la forme de fenêtres (7) sont prévus dans la paroi de la tige creuse.

10. Endoprothèse selon la revendication 9, caractérisée en ce que la cavité de la tige (1) est comblée de polyéthylène et que la surface de polyéthylène est revêtue d'apatite dans la région des fenêtres (7).

11. Endoprothèse selon la revendication 9, caractérisée en ce qu'une partie (11), qui reçoit ou présente une partie de l'articulation est assemblée de façon démontable à l'extrémité proche de l'articulation de la tige creuse (1) au moyen d'une bague de serrage (12) qui présente une amorce de rupture.

12. Endoprothèse selon une des revendications précédentes, caractérisée en ce que des dépressions (8 ou 9) sont prévues dans la paroi de la tige creuse (1) pour former des affaiblissements de la paroi.

13. Endoprothèse selon une des revendications précédentes, caractérisée en ce qu'au moins une partie de la surface est revêtue de matière Porocoat.

14. Endoprothèse selon les revendications 12 et 13, caractérisée en ce que les dépressions (9) dirigées vers l'extérieur sont comblées de matière Porocoat (10).

15. Endoprothèse selon la revendication 14, caractérisée en ce que les dépressions dirigées vers l'extérieur ne sont comblées que partiellement de matière Porocoat, de sorte que la surface de la dépression munie de la matière Porocoat forme un creux relativement aux régions adjacentes de la surface la tige de la prothèse.

16. Endoprothèse selon une des revendications précédentes, caractérisée en ce que la tige est conformée sans collerette.

17. Endoprothèse selon la revendication 11, caractérisée en ce que la partie d'articulation (11) présente un col d'emmanchement (6).
